# EUROPEAN PATENT SPECIFICATION

(11) **EP 3 378 945 B1**
(45) Date of publication and mention of the grant of the patent: **28.04.2021**
(21) Application number: 16876031.2
(22) Date of filing: 14.12.2016
(51) Int. Cl.: C12Q 1/04, G01N 33/50, C12N 11/02

(54) **METHOD FOR DISTINGUISHING PARTICULAR CELLS FROM MIXTURE OF HETEROGENEOUS CELLS**
VERFAHREN ZUR UNTERSCHEIDUNG VON EINZELNEN ZELLEN AUS EINER MISCHUNG AUS HETEROGENEN ZELLEN
PROCÉDÉ POUR DISTINGUER DES CELLULES PARTICULIÈRES D'UN MÉLANGE DE CELLULES HÉTÉROGÈNES

(30) Priority: 14.12.2015 KR 20150177852
(43) Date of publication of application: 26.09.2018
(73) Proprietor: Quanta Matrix Co., Ltd., Jongno-gu, Seoul 03082 (KR)
(72) Inventor: KWON, Sung Hoon, Seoul 06294 (KR); JUNG, Yong Gyun, Seoul 04313 (KR); CHOI, Jung Il, Seoul 08790 (KR); YOO, Jung Heon, Seoul 03082 (KR); KIM, Dong Young, Seongnam-si Gyeonggi-do 13597 (KR); JEONG, Hyun Yong, Seoul 07993 (KR); HAN, Sang Kwon, Seoul 08816 (KR)
(74) Representative: Prüfer & Partner mbB Patentanwälte · Rechtsanwälte
(86) International application number: PCT/KR2016/014671
(87) International publication number: WO 2017/105089

(56) References cited:
- WO-A2-2005/043121
- JP-A- 2008 522 165
- JP-B2- 3 531 196
- KR-A- 20000 071 045
- KR-A- 20140 016 896
- US-A1- 2013 196 364
- Jungil Choi: "RAPID ANTIBIOTIC SUSCEPTIBILITY TEST BASED ON THE MICROFLUIDIC AGAROSE CHANNEL WITH SINGLE CELL IMAGING PROCESS", Miniaturized Systems for Chemistry and Life Sciences, 1 November 2012 (2012-11-01), XP055507877, Retrieved from the Internet: URL:https://pdfs.semanticscholar.org/500f/ a37fda8361b7e804a92c5120cc2102c3f5bf.pdf [retrieved on 2018-09-18]
- JUNGIL CHOI ET AL: "Direct, rapid antimicrobial susceptibility test from positive blood cultures based on microscopic imaging analysis", SCIENTIFIC REPORTS, vol. 7, no. 1, 25 April 2017 (2017-04-25), XP055507839, DOI: 10.1038/s41598-017-01278-2

## Description

### Technical Field

The present disclosure relates to a method for distinguishing particular cells from a mixture of heterogeneous cells.

### Background Art

Water-based liquids containing mixtures of various types of cells are present in natural environments and humans. For example, a large number of microbial species are mixed in a water-based liquid sample taken from the river or sea. Human blood essentially includes blood cells consisting of erythrocytes, leukocytes, and thrombocytes. In a special case, human blood may further include circulating tumor cells¹ or erythrocytes infected with protozoa (e.g., malaria parasites), bacteria or viruses. Particular cells such as circulating tumor cells or bacterial cells need to be distinguished from various types of heterogeneous cells present in samples derived from natural environments and humans. This is essential to diagnose the state of the samples or understand the pathological conditions of the patients.

A molecular genetic method is used to determine whether particular cells are present in a sample containing a mixture of heterogeneous cells. This method is carried out by constructing primers amplifying a gene present in only particular cells and performing polymerase chain reaction (PCR) to determine the presence or absence of the particular cells². Another method for determining the presence of particular cells is carried out by spreading a sample containing a mixture of heterogeneous cells on slide glass, selectively staining particular cells, and observing the particular cells with a microscope³. A flow cytometric method is also known based on the use of a fluorescent material specifically attached to the surface of particular cells⁴. An alternative method exists based on a difference in size, density or degree of deformation between cells in a microfluid⁵. However, both the molecular genetic method and the spreading-based method are carried out after cell death and require the use of expensive PCR equipment and high-magnification microscopes. The flow cytometric method also requires an expensive analytical instrument. The method based on a difference in size, density or degree of deformation between cells in a microfluid has a critical limitation in that its use is not allowed when the difference is insignificant. Thus, there exists a need for a more efficient method for determining the presence of particular cells in a sample containing a mixture of heterogeneous cells.

Patent document 1⁶ discloses a rapid antibiotic susceptibility testing system based on bacterial immobilization using gelling agent, antibiotic diffusion and tracking of single bacterial cells. The testing method includes: providing a mixture solution of a gelling agent (agarose) and a sample comprising microbes (complex samples such as biofilms) to a gelling device; solidifying the mixture solution to form a solid thin film in which the microbe is immobilized; supplying a bioactive agent to the solid thin film and allowing the bioactive agent to diffuse into the solid thin film; and microscopic imaging (time-lapse) the individual responses of the single microbial cells and formed colonies to the bioactive agent, and determining the minimum inhibitory concentration (MIC) of the bioactive agent based on the analysis of the images to obtain AST results.

J. Choi (2012)⁷ describes a microfluidic channel system for Antibiotics Susceptibility Test (AST) to reduce both amount of bacteria and test time dramatically. The agarose based microfluidic channel system fixes bacterial cell and tracks single cell growth to reduce AST assay time and to determine MICs of antibiotics. In this system, the conventional laboratory bacteria and four standard bacteria were tested with several kinds of antibiotics to determine the MIC values.

Patent document 2⁸ describes a method for detecting the presence of microorganisms in clinical and non-clinical specimens using a detectable metabolic change which occurs depending an the presence or growth of a micro-organism.

### [References]

1. V. Plaks, C. D. Koopman, Z. Werb, Circulating Tumor Cells, Science, Vol. 341, No. 6151, p. 1186-1188, 2013.
2. A. Moody, Rapid Diagnostic Tests for Malaria Parasites, Clinical Microbiology Reviews, Vol. 15, No. 1, p. 66-78, 2002.
3. M. A. Jensen, J. A. Webster, N. Straus, Rapid Identification of Bacteria on the Basis of Polymerase Chain Reaction-Amplified Ribosomal DNA Spacer Polymorphisms, Applied and Environmental Microbiology, Vol. 59, No. 4, p. 945-952, 1993.
4. K. Czechowska, D. R. Johnson, J. R. van der Meer, Use of Flow Cytometric Methods for Single-Cell Analysis in Environmental Microbiology, Current Opinion in Microbiology, Vol. 11, No. 3, p. 205-212, 2008.
5. H. T. K. Tse, D. R. Gossett, Y. S. Moon, M. Masaeli, M. Sohsman, Y. Ying, K. Mislick, R. P. Adams, J. Rao, D. Di Carlo, Quantitative Diagnosis of Malignant Pleural Effusions by Single-Cell Mechanophenotyping, Science Translational Medicine, Vol. 5, No. 212, p. 212ra163, 2013.
6. Patent document 1: US 2013/196364 A1
7. Jungil Choi: "Rapid Antibiotic Susceptibility Test Based on the Microfluidic Agarose Channel With Single Cell Imaging Process", Miniaturized Systems for Chemistry and Life Sciences, 1 November 2012
8. Patent document 2: KR 2000 0071045 A1

### Detailed Description of the Invention

### Means for Solving the Problems

According to one aspect of the present invention, there is provided a method for distinguishing particular cells as set forth in claim 1. Preferred embodiments are set out in the dependent claims.

Accordingly, the present invention provides a method for distinguishing particular cells from a mixture of heterogeneous cells in a blood sample, wherein the particular cells comprise bacteria, the method comprising:
(a) providing a water-based liquid blood sample containing a mixture of heterogeneous cells;
(b) collecting at least a portion of the sample, introducing the collected portion of the sample into a porous medium, and immobilizing the heterogeneous cells in the porous medium;
(c) applying a first bioactive stimulus to the porous medium to cause division of the bacteria only as said particular cells, and additionally applying another second type of bioactive stimulus to the porous medium in step (c); and
(d) distinguishing the particular cells from the other types of cells through microscopic imaging,
wherein culture or purification of the heterogeneous cells is omitted after the provision of the sample and before the application of the first bioactive stimulus,
wherein a nutrient medium for growth of the bacterial cells is applied as the first bioactive stimulus to selectively grow the bacterial cell only but not blood cells,
wherein a drug for preventing the growth of the particular cells is applied as the other second type of bioactive stimulus, simultaneously with or after the application of the first bioactive stimulus, and
wherein the particular cells are distinguished by observing microcolonies formed as a result of division of the particular bacterial cells when present.

### Brief Description of the Drawings

Fig. 1 shows a sample containing a mixture of heterogeneous cells according to one embodiment of the present invention.
Fig. 2 shows heterogeneous cells immobilized in a porous medium according to one embodiment of the present invention.
Fig. 3 shows the application of a selective bioactive stimulus to which only particular cells respond according to one embodiment of the present invention.
Fig. 4 shows microscopic observation of microcolonies of particular cells cultured after the application of a selective stimulus according to one embodiment of the present invention.
Fig. 5 shows the results of microscopic observation before and after a selective bioactive stimulus was applied to a blood culture broth containing a mixture of *S. aureus* bacteria and blood cells.

### Mode for Carrying out the Invention

The present inventors succeeded in developing a method for distinguishing particular cells from a blood sample containing various types of cells derived from a living organism, in particular from human.

The method for distinguishing particular cells from a mixture of heterogeneous cells includes, among others, immobilizing the heterogeneous cells in a porous material, wherein the particular cells comprise bacteria, applying a first bioactive stimulus to the porous material to selectively grow the the bacteria as said particular cells of interest, and observing microcolonies of the particular cells with a microscope.

When a sample containing heterogeneous cells is immobilized in a porous medium, particular cells are initially impossible to distinguish because the properties and shapes of the cells are not discernable. When the bioactive stimulus is applied, the particular cells start to divide to form microcolonies, which are discernable from other background cells and can be visually observed with a suitable instrument, such as a microscope.

In the present invention, the sample is a blood sample containing bacteria. The bacteria are initially difficult to distinguish from blood cells because the bacteria have a size of 7 to 15 µm and the blood cells have a size of 1 to 2 µm. However, when a medium causing cell division of the bacteria only is introduced, the bacteria only divide once every 30 minutes on average and form cell colonies larger than the blood cells after the lapse of several hours, with the result that the bacteria can be visually observed.

Exemplary embodiments of the present invention will now be described in more detail with reference to the accompanying drawings.

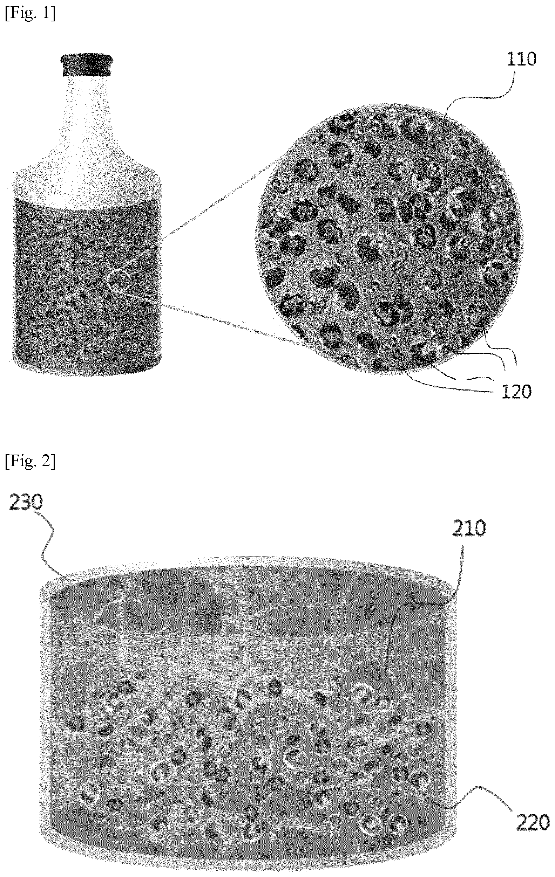

Fig. 1 shows a sample containing a mixture of heterogeneous cells according to one embodiment of the present invention. The sample may be provided in the form of a water-based liquid 110. The heterogeneous cells 120 may be either prokaryotic or eukaryotic cells or both. Examples of the heterogeneous cells include mammalian cells, tumor cells, blood cells, prokaryotes (bacteria including cyanobacteria and archaea), filamentous fungi, yeasts, myxomycetes, basidiomycetes, unicellular algae, and protozoa. The sample may include pathogenic microorganisms. Examples of the pathogenic microorganisms include bacteria such as micrococcus, bacillus, spirillum, and actinomyces bacteria. The sample including the heterogeneous cells 120 may be derived from a living organism, such as a human, or an animal. The heterogeneous cells 120 may include cells from blood, pus, and secretions.

Fig. 2 shows the heterogeneous cells 220 immobilized in the porous medium 210. The porous medium 210 including the heterogeneous cells 220 is a liquid material that can be gelled. That is, the heterogeneous cells 220 are dispersed in the liquid medium in a container 230 such as a microwell and are then immobilized in the liquid medium when the liquid medium is gelled under appropriate physical or chemical environmental conditions.

For example, the liquid medium may include at least about 95% of water and may be gelled in the presence of a gelling agent. For example, the gelling agent may be used in an amount of 0.5 to 4% by weight, based on the weight of the liquid medium. Preferably, the gelling agent is agar or agarose.

The porous medium 210 may be made of a hydrogel or porous membrane. The hydrogel may be a natural or synthetic polymeric material. Examples of such hydrogels include collagen, fibrin, agarose, agar, matrigel, alginate, and gelatin.

The porous medium 210 is initially a liquid and may be gelled under various conditions, including temperature, light, pH, pressure, and vibration conditions. For example, the porous medium is a liquid at a high temperature and may be gelled at a low temperature.

The sample taken from a living organism is not cultured or purified before application of the first bioactive stimulus. Particularly, the sample may be gelled by mixing with a gelling agent without the need for pure culture. The sample may be, for example, a positive blood culture sample. In this case, various types of blood cells together with bacteria may be immobilized in the porous medium. Pure culture of the sample requiring about 16-24 hours after sampling is omitted, which greatly reduces the time to detect the particular cells.

The sample taken from a living organism would require a culture time of ~1-2 days to increase the number of bacteria, however this process is omitted when the concentration of the particular cells in the sample is sufficiently high to observe the particular cells. When the heterogeneous cells 220 are immobilized in the porous medium 210, a desired concentration of the particular cells may be, for example, in the range of 10³ to 10⁵ CFU/ml. Within this range, the particular cells are easy to observe in the subsequent step.

In this case, desired results can be obtained within several hours after sampling, greatly shortening the time for analysis.

The various types of cells in the sample may be introduced into and immobilized in the pores of the gelled porous medium 210. That is, the heterogeneous cells 220 dispersed in the liquid porous medium 210 before gelling are three-dimensionally immobilized in the matrix of the porous medium 210 after gelling. As a result, the motile cells in the liquid medium are fixed at particular locations after gelling. This fixing facilitates microscopic observation of changes of the cells at and around the fixed locations in observation areas.

The pores of the porous medium 210 may have a size of one nanometer to several millimeters. The presence of the pores facilitates the immobilization of the various types of heterogeneous cells 220. Taking into consideration the size of the cells, the pore size is preferably from 1 to 100 µm.

The thickness of the porous matrix taking the form of the gelled porous medium 210 is not especially limited as long as colonies of the immobilized heterogeneous cells can be observed with a microscope. For example, the porous matrix may have a thickness ranging from several micrometers to several millimeters. The method may further include supplying a culture medium to the mixture of the sample and the gelling agent and culturing the cells.

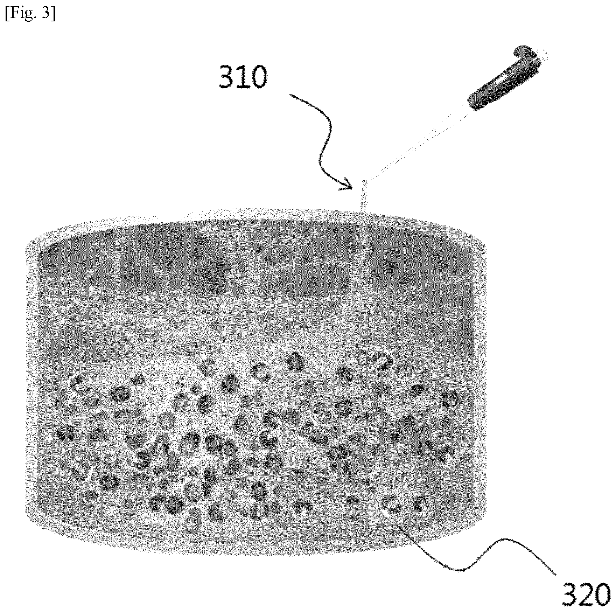

Fig. 3 shows the application of the selective first bioactive stimulus to which only particular bacterial cells respond. The selective bioactive stimulus 310 includes media for selective growth of particular bacterial cells. The stimulus 310 may be applied continuously. Alternatively, the stimulus 310 may be applied intermittently at predetermined time intervals. The stimulus 310 may be applied to some or all of the heterogeneous cells in the sample. Preferably, the stimulus 310 is applied to all of the heterogeneous cells. The bioactive stimulus 310 allows selective growth of the particular cells 320.

Another second type of bioactive stimulus is additionally applied to the porous medium. The additional bioactive stimulus may be applied simultaneously with or after the application of the first stimulus. A nutrient medium for growth of the particular cells is applied as the first bioactive stimulus, and at the same time or thereafter, a drug for preventing the growth of the particular cells is applied as the additional, second bioactive stimulus. Thereby, the growth sensitivity of the particular cells to the drug can be examined.

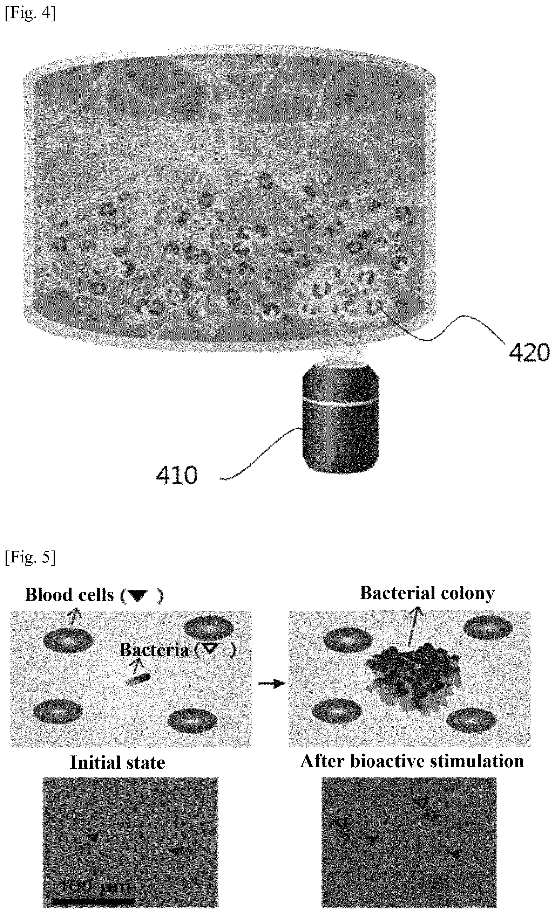

Fig. 4 shows microscopic observation of microcolonies of the particular cells cultured after the application of the selective, first bioactive stimulus.

The selective bioactive stimulus causes cell division. The immobilized cells divide to form colonies larger than their initial size. The cells having selectively responded to the bioactive stimulus can be distinguished by their size variation when observed with a microscope 410.

That is, the particular cells can be distinguished by observing the microcolonies 420 formed as a result of division of the particular cells. The microscopic imaging is performed by taking images of the microcolonies 420 of the target cells at an observable magnification. Considering the typical size (1-2 µm) of bacteria, several tens to several hundreds of bacteria should gather to reach an observable size. The observable size of the microcolonies 420 is at least 10 µm, preferably at least 20 µm, more preferably at least 30 µm, even more preferably at least 50 µm. Considering the time for the microcolonies 420 to grow to the observable size, the particular cells can be rapidly distinguished within a short time, typically ≥2 hours, preferably 3 to 5 hours.

The presence of the microcolonies 420 can be observed in the bright or dark field mode, preferably in the bright field mode. Time-series changes in the growth and division of the cells can be tracked by time-lapse imaging of the entire area because the cells are spatially immobilized in the porous medium.

The microscopic imaging may be performed by time-lapse imaging of the target region during culture to track time-series changes in the area occupied by the heterogeneous cells present in the target region. The imaging procedure is carried out by taking images using a charge coupled device (CCD) or complementary metal oxide semiconductor (CMOS) camera when observed with the microscope 410 and converting the images into digital data using a coded program.

The method of the present invention is helpful in rapidly determining the presence or absence of bacteria in a blood sample from a patient and prescribing a drug for the patient.

The present invention will be explained in more detail with reference to the following examples. However, these examples are not limiting.

### [EXAMPLES]

### Experiment for distinguishing bacteria from blood sample infected with the bacteria

Blood from a patient infected with a *S*. *aureus* strain was cultured and sampled. The sample was mixed with liquid agarose (UltraPure agarose, Invitrogen) and injected into a 96-well microplate to immobilize the bacteria and the blood cells in the porous material. The porous structure of the agarose was formed by gelling at < 36 °C before use. Specifically, immediately after the culture sample containing the mixture of the bacteria and the blood cells was mixed with 0.5% agarose in a volume ratio of 1:400 at 45 °C, the resulting mixture was injected into a microwell plate and gelled at 25 °C for < 1 min. A liquid cation-adjusted Mueller-Hinton broth (CAMHB) as a bacterial culture broth for selective growth of the bacterial cells was supplied to the wells containing the gelled agarose. Immediately after the supply of CAMHB, the interior of the agarose was observed with a microscope and the microwell plate was incubated at 37 °C. 4 h after the incubation, the interior of the agarose was again observed with the microscope. For microscopic observation, a CCD camera was used to obtain images. The images were processed to track the growth of the bacterial cells.

The image data was converted into digital data using a C++-coded program. The raw image data in RGB format was converted into gray format and then the background data was erased to obtain noise-free clear data. Next, the images were rendered binary by increasing the contrast. A C++ program was used to detect different areas between the initial images and the images obtained 4 h after culture. New objects detected in areas where the initial objects had not been detected or objects detected with a size larger than the initial objects were recognized as bacterial cells.

Fig. 5 shows the results of microscopic observation before and after the selective bioactive stimulus was applied to the blood culture broth containing the mixture of the *S*. *aureus* strain and the blood cells. Referring to Fig. 5, the immobilization of the bacteria and the blood cells in the agarose and the growth of the bacteria were tracked.

After the *S. aureus* bacteria and the blood cells immobilized in the agarose were treated with the bacterial culture broth, the blood cells did not grow but the *S. aureus* grew and divided to form colonies, which were observed with a microscope. The presence of the *S. aureus* bacteria in the blood culture broth was rapidly distinguished from the heterogeneous cells within 4 h.

## Claims

1. A method for distinguishing particular cells from a mixture of heterogeneous cells in a blood sample, wherein the particular cells comprise bacteria, the method comprising: (a) providing a water-based liquid blood sample containing a mixture of heterogeneous cells;
(b) collecting at least a portion of the sample, introducing the collected portion of the sample into a porous medium, and immobilizing the heterogeneous cells in the porous medium;
(c) applying a first bioactive stimulus to the porous medium to cause division of the bacteria only as said particular cells, and additionally applying another second type of bioactive stimulus to the porous medium in step (c); and
(d) distinguishing the particular cells from the other types of cells through microscopic imaging,
wherein culture or purification of the heterogeneous cells is omitted after the provision of the sample and before the application of the first bioactive stimulus,
wherein a nutrient medium for growth of the bacterial cells is applied as the first bioactive stimulus to selectively grow the bacterial cell only but not blood cells,
wherein a drug for preventing the growth of the particular cells is applied as the other second type of bioactive stimulus, simultaneously with or after the application of the first bioactive stimulus, and
wherein the particular cells are distinguished by observing microcolonies formed as a result of division of the particular bacterial cells when present.

2. The method according to claim 1, wherein the heterogeneous cells are derived from a living organism.

3. The method according to claim 1, wherein the heterogeneous cells dispersed in the liquid porous medium before gelling are three-dimensionally immobilized in the matrix of the porous medium after gelling.

4. The method according to claim 1, wherein the microscopic imaging is performed by taking images of the microcolonies at an observable magnification.

5. The method according to claim 1, wherein the microscopic imaging is performed by time-lapse imaging of the target region during culture to track time-series changes in the area occupied by the heterogeneous cells present in the target region.

## Patentansprüche

1. Ein Verfahren zum Unterscheiden von bestimmten Zellen aus einer Mischung von heterogenen Zellen in einer Blutprobe, wobei die bestimmten Zellen Bakterien umfassen, wobei das Verfahren umfasst: (a) Bereitstellen einer ein Gemisch heterogener Zellen enthaltenden flüssigen Blutprobe auf Wasserbasis;
(b) Aufnehmen mindestens eines Teils der Probe, Einbringen des aufgenommenen Teils der Probe in ein poröses Medium und Immobilisieren der heterogenen Zellen in dem porösen Medium;
(c) Anwenden eines ersten bioaktiven Stimulus auf das poröse Medium, um nur die Teilung der Bakterien als die bestimmten Zellen zu bewirken, und zusätzlich Anwenden einer anderen, zweiten Art eines bioaktiven Stimulus auf das poröse Medium in Schritt (c); und
(d) Unterscheiden der bestimmten Zellen von den anderen Arten von Zellen durch mikroskopische Bildgebung,
wobei die Kultur oder Reinigung der heterogenen Zellen nach dem Bereitstellen der Probe und vor dem Anwenden des ersten bioaktiven Stimulus unterlassen wird,
wobei ein Nährmedium zum Wachstum der Bakterienzellen als erster bioaktiver Stimulus angewandt wird, um selektiv nur die Bakterienzelle, nicht aber Blutzellen wachsen zu lassen, wobei ein Arzneistoff zum Verhindern des Wachstums der bestimmten Zellen als die andere, zweite Art von bioaktivem Stimulus gleichzeitig mit oder nach der Anwendung des ersten bioaktiven Stimulus angewandt wird, und
wobei die bestimmten Zellen durch Beobachtung von Mikrokolonien unterschieden werden, die, falls vorhanden, als Ergebnis der Teilung der bestimmten Bakterienzellen gebildet werden.

2. Das Verfahren gemäß Anspruch 1, wobei die heterogenen Zellen von einem lebenden Organismus abgeleitet sind.

3. Das Verfahren gemäß Anspruch 1, wobei die heterogenen Zellen, die vor einem Gelieren in dem flüssigen porösen Medium dispergiert werden, nach dem Gelieren dreidimensional in der Matrix des porösen Mediums immobilisiert werden.

4. Das Verfahren gemäß Anspruch 1, wobei die mikroskopische Bildgebung durch Aufnahme von Bildern der Mikrokolonien bei einer beobachtbaren Vergrößerung durchgeführt wird.

5. Das Verfahren gemäß Anspruch 1, wobei die mikroskopische Bildgebung durch Zeitrafferaufnahmen der Zielregion während der Kultur durchgeführt wird, um Veränderungen in der Fläche, die von den in der Zielregion vorhandenen heterogenen Zellen eingenommen wird, im Zeitverlauf zu verfolgen.

## Revendications

1. Procédé pour distinguer des cellules particulières provenant d'un mélange de cellules hétérogènes dans un échantillon de sang, dans lequel les cellules particulières comprennent des bactéries, le procédé comprenant :
(a) la fourniture d'un échantillon de sang liquide à base d'eau contenant un mélange de cellules hétérogènes ;
(b) la collecte d'au moins une partie de l'échantillon, l'introduction de la partie collectée de l'échantillon dans un milieu poreux, et l'immobilisation des cellules hétérogènes dans le milieu poreux ;
(c) l'application d'un premier stimulus bioactif au milieu poreux pour provoquer seulement la division des bactéries en tant que lesdites cellules particulières, et en outre l'application d'un autre second type de stimulus bioactif au milieu poreux dans l'étape (c) ; et
(d) la distinction des cellules particulières par rapport aux autres types de cellules par imagerie microscopique,
dans lequel une mise en culture ou une purification des cellules hétérogènes est omise après la fourniture de l'échantillon et avant l'application du premier stimulus bioactif,
dans lequel un milieu nutritif pour la croissance des cellules bactériennes est appliqué en tant que premier stimulus bioactif pour sélectivement faire croître seulement la cellule bactérienne mais pas les cellules sanguines,
dans lequel un médicament pour empêcher la croissance des cellules particulières est appliqué en tant que l'autre seconde type de stimulus bioactif, en même temps que ou après l'application du premier stimulus bioactif, et
dans lequel les cellules particulières sont distinguées en observant des microcolonies formées en raison de la division des cellules bactériennes particulières quand elles sont présentes.

2. Procédé selon la revendication 1, dans lequel les cellules hétérogènes sont dérivées d'un organisme vivant.

3. Procédé selon la revendication 1, dans lequel les cellules hétérogènes dispersées dans le milieu poreux liquide avant gélification sont immobilisées de manière tridimensionnelle dans la matrice du milieu poreux après gélification.

4. Procédé selon la revendication 1, dans lequel l'imagerie microscopique est effectuée en prenant des images des microcolonies à un grossissement observable.

5. Procédé selon la revendication 1, dans lequel l'imagerie microscopique est effectuée par imagerie à laps de temps de la région cible pendant une culture pour suivre des changements de séries chronologiques dans la zone occupée par les cellules hétérogènes présentes dans la région cible.
